# EUROPEAN PATENT SPECIFICATION

(11) **EP 1 128 837 B1**
(45) Date of publication and mention of the grant of the patent: **26.02.2003**
(21) Application number: 99972104.6
(22) Date of filing: 15.11.1999
(51) Int. Cl.: A61K 35/14, A61P 9/10

(54) **MEDICAMENT FOR PREVENTING AND REVERSING ATHEROSCLEROSIS IN MAMMALS**
ARZNEIMITTEL ZUR VERMEIDUNG UND UMKEHRUNG DER ATHEROSKLEROSE BEI SÄUGETIEREN
TECHNIQUE DE PREVENTION ET FAVORISANT LA REGRESSION DE L'ATHEROSCLEROSE CHEZ LES MAMMIFERES

(30) Priority: 13.11.1998 US 190236; 06.05.1999 US 304262
(43) Date of publication of application: 05.09.2001
(73) Proprietor: Vasogen Ireland Limited, Shannon, County Clare (IE)
(72) Inventor: STEWART, Duncan, J., Toronto, Ontario M4P 2K2 (CA)
(74) Representative: Gowshall, Jonathan Vallance
(86) International application number: CA9901092
(87) International publication number: WO00029003

(56) References cited:
- WO-A-93/15778
- WO-A-98/07436
- US-A- 4 831 268

## Description

### FIELD OF THE INVENTION

This invention relates to the use of treated, modified blood in connection with certain abnormal mammalian physical conditions and disease states. More specifically, it relates to modified mammalian blood and its use in the treatment of atherosclerosis and cardiovascular disorders associated therewith or resulting therefrom.

### BACKGROUND OF THE INVENTION

Atherosclerosis, the build-up of fatty plaque deposits within the walls of blood vessels, is the cause of most types of cardiovascular disease. The main mechanism by which atherosclerosis leads to morbidity and mortality is by production of atherosclerotic plaques which may cause significant vessel obstruction but which more importantly are the site of the thrombus formation which causes heart attacks and most strokes.

The pathogenesis of atherosclerosis is complex and is associated with a number of risk factors, including hyperlipidemia, hypertension, obesity, smoking, diabetes and stress. Hyperlipidemias such as hypercholesterolemia and elevated serum triglyceride levels are among the most potent risk factors in the causation of atherosclerosis. For example, high levels of serum cholesterol bound to low density lipoprotein (LDL), intermediate density lipoprotein (IDL) or very low density lipoprotein (VLDL) are known to correlate strongly with the occurrence of atherosclerosis in humans. As well, low levels of high density lipoproteins (HDL) are a known atherosclerosis risk factor. In particular, it is believed that the higher the circulating levels of cholesterol in the form of LDL, IDL and VLDL cholesterol, and the higher the circulating levels of other lipids such as triglycerides, the more likely it is that cholesterol and lipids will be deposited within the blood vessel wall and cause or contribute to atherosclerosis. However, it is important to note that less than 50% of heart attack patients have elevated blood lipid levels from which it can be inferred that other factors including dysfunction of the vascular endothelium are important in the pathogenesis of atherosclerosis.

It is believed that exposure to these risk factors can result in localized damage to the endothelial cells that line blood vessel walls. Endothelial cells exposed to injury become "sticky" through up-regulation of adhesion molecules, which causes leucocytes to adhere and migrate through the endothelium to the tissues, a process accompanied by the release of inflammatory cytokines and other inflammatory mediators that cause further endothelial dysfunction making the endothelium more permeable to the lipid particles. This process is believed to contribute to the gradual build-up of plaque deposits in the blood vessel wall, resulting in narrowing of the lumen of the vessel, impaired blood flow and thrombus formation.

Current therapies for atherosclerosis consist mainly of life style changes including risk factor modification, diet (low fat), and lipid lowering drugs which in the case of both hyper and normolipidic patients have been shown to significantly reduce the incidence of subsequent cardiac and cerebrovascular events. As well, these therapies have been shown to retard progression of atherosclerosis and to some extent cause regression of the plaques. However, it is clear that further measures targeted directly at improving endothelial function and/or inhibiting the inflammatory processes that drive the disease which will be equally or more efficacious are required.

It is also important to note that plaque stability, based on plaque architecture and lipid content as well as quality of endothelial function, is the key factor in determining the tendency towards the thrombus formation which in turn usually leads to tissue infarction. Thus, in addition to the lipid lowering agents, therapies which improve endothelial function will not only inhibit progression and cause regression of atherosclerosis but will also reduce cardiovascular event rates.

U.S. Patent No. 4,831,268 to Fisch et al. provides a method for the radiation of corporeal blood to prevent atherosclerosis related heart and vascular diseases caused by disturbances in the fat exchange. The disclosed process involves irradiating the blood in a blood conducting tube with radiation having an intensity of from about 1 mWcm⁻² to 10 mWcm⁻² in a wavelength range of from about 320 nm to 600 nm.

International Publication No. WO 93/15778 to Bolton provides a method for treating blood to prevent aggregation of blood platelets in a human patient. The method disclosed by Bolton comprises contacting an aliquot of blood with a blood platelet-aggregation inhibiting amount of ozone gas and ultraviolet radiation.

Bolton discloses that such treated blood may be used in the treatment of peripheral vascular disease, thrombotic diseases such as coronary thrombosis and pulmonary thrombosis, stroke, eclampsia and pre-eclampsia.

### SUMMARY OF THE INVENTION

The present invention overcomes at least some of the above-noted and other disadvantages of presently known therapies for treatment of cardiovascular disease, particularly atherosclerosis, by making possible a method for preventing and causing regression of atherosclerosis in which an aliquot of mammalian blood is treated ex vivo and subsequently introduced into the body of a mammalian subject.

The aliquot of blood is treated by being subjected to two or more stressors which have been found to modify the blood. According to the present invention, the blood aliquot can be modified by subjecting the blood, or separated cellular or non-cellular fractions of the blood, or mixtures of the separated cells and/or non-cellular fractions of the blood, to stressors selected from temperature stressors, electromagnetic emissions and oxidative environments, or any combination of such stressors, simultaneously or sequentially.

The observed effects of the modified blood or blood components of the present invention, when introduced into the mammalian subject's body, are several in number. Firstly, mammalian subjects treated according to the present invention show substantially reduced development of plaque as reflected by reduced deposition of lipids within blood vessel walls, as compared to untreated subjects. As well as retarding the progression of plaque deposition, the use of the invention has been shown to cause existing plaques to regress. Specifically, reductions in plaque deposition of as high as about 75 percent have been observed in subjects treated by the medicament prepared according to the invention.

Secondly, there has been observed a reduction in total serum cholesterol levels in some experiments, primarily due to a reduction in the levels of LDL and VLDL cholesterol. Levels of beneficial HDL cholesterol are not reduced. Reductions in cholesterol levels of as high as about 40 percent, as compared to subjects which received untreated blood as a placebo, have been observed. Thirdly, there has also been observed a reduction in serum triglyceride levels. Such reductions in serum cholesterol and triglycerides would be expected to delay the onset and retard the progression of atherosclerosis due to hyperlipidemia.

It is believed that the beneficial effect of the use according to the present invention is related to one or more of a direct effect on the endothelium rendering it more resistant to lipid deposition or a reduction in the inflammatory response to the oxidized lipid. A reduction in lipid levels, which is not always observed when subjects are treated according to the invention, could still play a role in reducing atherosclerosis.

The applicant believes that blood or blood components, modified according to the present invention, when introduced into a subject's body, has the effect of down-regulating the inflammatory immune responses involved in atherogenesis. Specifically, the applicant believes that blood modified according to the invention down-regulates the activity of the TH1 component of the T-cells present in the blood, responsible for secretion of inflammatory cytokines such as IL-2, IL-6, IL-8, IFN-γ and TNF-α, and/or up-regulates the TH-2 component of T-cells responsible for secretion of anti-inflammatory cytokines, for example IL-10 and IL-4. It is also believed that the medical use claimed may result in an inhibition of the activation state of circulating leucocytes, a reduction in the stickiness of the endothelium, or a combination of both, thus reducing endothelial injury. Furthermore, by down-regulating the inflammatory immune response, it is believed that the claimed medical use has the effect of stabilizing atherosclerotic plaques, which would be expected to reduce the incidence of thrombus formation.

Although the above mechanism may explain how the claimed use affects atherogenesis, it does not completely explain the observation that it achieves significant regression of existing plaque deposits.

Accordingly, in one aspect the present invention provides a medicement for use in preventing progression of atherosclerosis in a mammal. comprising an aliquot of mammalian blood which has been subjected extracorporeally to an electromagnetic emission and an oxidative environment.

### BRIEF DESCRIPTION OF THE DRAWINGS

The invention will now be more fully described, by way of example only, with reference to the accompanying drawings, in which:

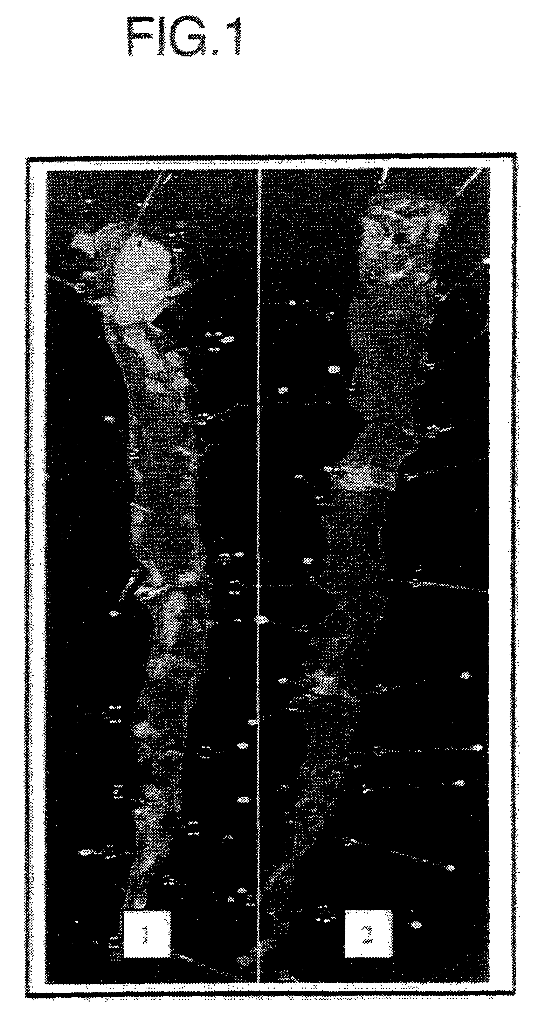

Figure 1 comprises photographs of two full length aortae obtained from LDL receptor deficient mice which underwent the study described in Example 1, the aorta labeled 1 being obtained from an animal which received a high cholesterol diet and sham treatments, and the aorta labeled 2 being obtained from an animal which received a high cholesterol diet and was treated according to a preferred method of the present invention, with aortic lipid deposition being made visible by staining the aortae with oil red O; and

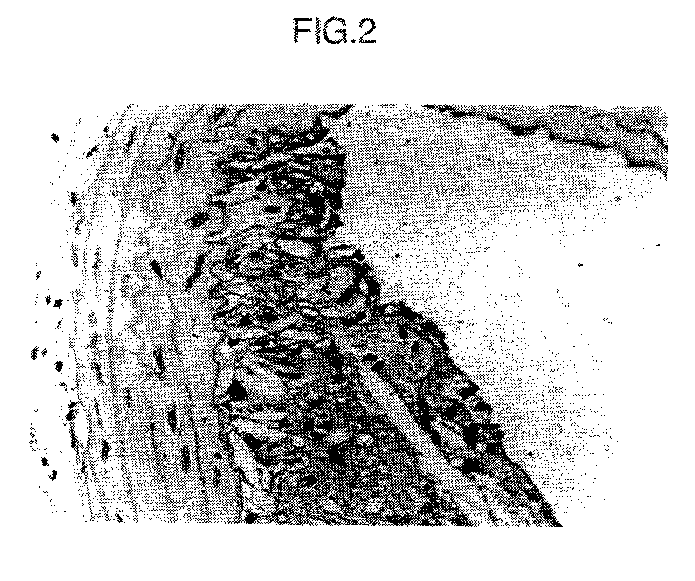

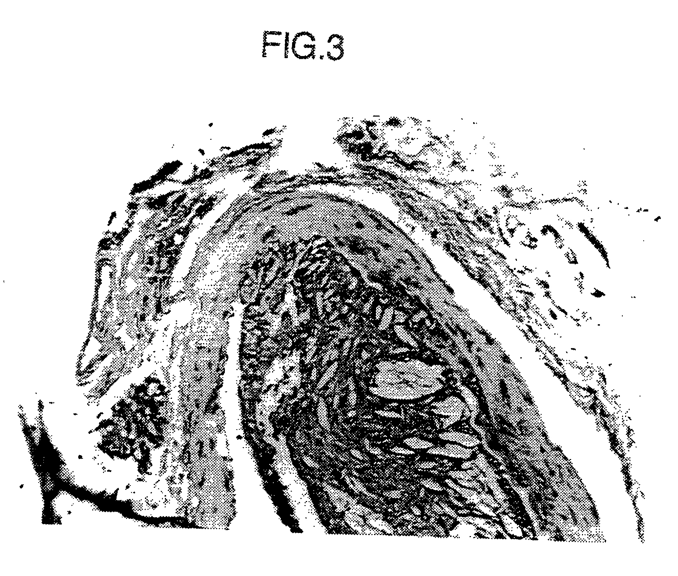

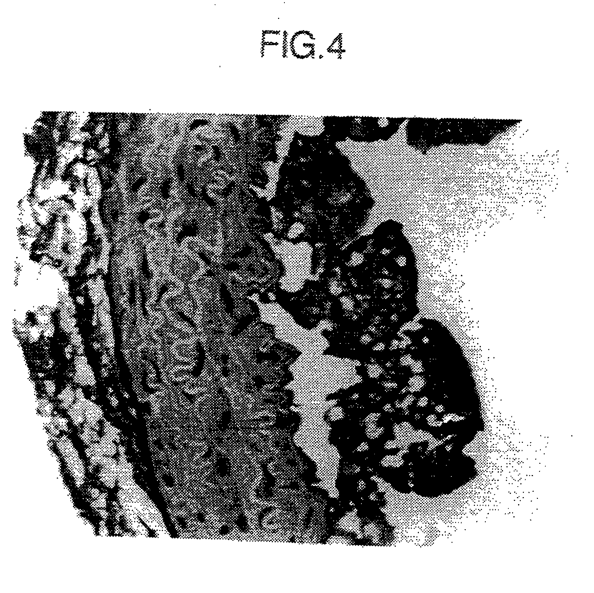

Figures 2 to 4 comprise photographs of aortic cross-sections obtained from LDL receptor deficient mice which underwent the study described in Example 1, the aorta shown in Figure 2 being obtained from an animal which received a normal diet, the aorta of Figure 3 being obtained from an animal which received the high cholesterol diet and sham treatments, and the aorta of Figure 4 being obtained from an animal which received a high cholesterol diet and was treated according to a preferred method of the present invention, with the presence of macrophages in the aortic plaque deposits being made visible by immunostaining the aortae.

### DETAILED DESCRIPTION OF PREFERRED EMBODIMENTS

According to a preferred process of the present invention, an aliquot of blood is extracted from a mammalian subject, preferably a human, and the aliquot of blood is treated ex vivo with certain stressors, described in more detail below. The terms "aliquot", "aliquot of blood" or similar terms used herein include whole blood, separated cellular fractions of the blood including platelets, separated non-cellular fractions of the blood including plasma, and combinations thereof. The effect of the stressors is to modify the blood, and/or the cellular or non-cellular fractions thereof, contained in the aliquot. The modified aliquot can be then re-introduced into the subject's body by any method suitable for vaccination, preferably selected from intra-arterial injection, intramuscular injection, intravenous injection, subcutaneous injection, intraperitoneal injection, and oral, nasal or rectal administration.

The stressors to which the aliquot of blood is subjected ex vivo according to the method of the present invention are selected from an oxidative environment and an electromagnetic emission, optionally in combination with temperature stress (blood temperature above or below body temperature), simultaneously or sequentially. Suitably, in human subjects, the aliquot has a volume sufficient that, when re-introduced into the subject's body, a reduction in a serum lipid levels, a retardation in progression or a regression of atherosclerotic plaque formation, and/or increased stability of atherosclerotic plaque is achieved in the subject. Preferably, the volume of the aliquot is up to about 400 ml, preferably from about 0.1 to about 100 ml, more preferably from about 5 to about 15 ml, even more preferably from about 8 to about 12 ml, and most preferably about 10 ml.

It is preferred, according to the invention, to apply all three of the aforementioned stressors simultaneously to the aliquot under treatment, in order to ensure the appropriate modification to the blood. Care must be taken to utilize an appropriate level of the stressors to thereby effectively modify the blood to achieve a significant reduction in atherosclerotic plaque in the subject or to achieve stabilization of atherosclerotic plaque in the subject.

The temperature stressor warms the aliquot being treated to a temperature above normal body temperature or cools the aliquot below normal body temperature. The temperature is selected so that the temperature stressor does not cause excessive hemolysis in the blood contained in the aliquot and so that, when the treated aliquot is injected into a subject, a lipid reduction and/or a retardation in progression or regression in the formation of atherosclerotic plaque will be achieved. Preferably, the temperature stressor is applied so that the temperature of all or a part of the aliquot is up to about 55°C, and more preferably in the range of from about -5°C to about 55°C.

In some preferred embodiments of the invention, the temperature of the aliquot is raised above normal body temperature, such that the mean temperature of the aliquot does not exceed a temperature of about 55°C, more preferably from about 40°C to about 50°C, even more preferably from about 40°C to about 44°C, and most preferably about 42.5 ± 1 °C.

In other preferred embodiments, the aliquot is cooled below normal body temperature such that the mean temperature of the aliquot is within the range of from about -5°C to about 36.5°C, even more preferably from about 10°C to about 30°C, and even more preferably from about 15°C to about 25°C.

The oxidative environment stressor can be the application to the aliquot of solid, liquid or gaseous oxidizing agents. Preferably, it involves exposing the aliquot to a mixture of medical grade oxygen and ozone gas, most preferably by bubbling through the aliquot, at the aforementioned temperature range, a stream of medical grade oxygen gas having ozone as a minor component therein. The ozone content of the gas stream and the flow rate of the gas stream are preferably selected such that the amount of ozone introduced to the blood aliquot, either on its own or in combination with other stressors, does not give rise to excessive levels of cell damage such that the therapy is rendered ineffective. Suitably, the gas stream has an ozone content of up to about 300 µg/ml, preferably up to about 100 µg/ml, more preferably about 30 µg/ml, even more preferably up to about 20 µg/ml, particularly preferably from about 10 µg/ml to about 20 µg/ml, and most preferably about 14.5 ± 1.0 µg/ml. The gas stream is suitably supplied to the aliquot at a rate of up to about 2.0 litres/min, preferably up to about 0.5 litres/min, more preferably up to about 0.4 litres/min, even more preferably up to about 0.33 litres/min, and most preferably about 0.24 ± 0.024 litres/min. The lower limit of the flow rate of the gas stream is preferably not lower than 0.01 litres/min, more preferably not lower than 0.1 litres/min, and even more preferably not lower than 0.2 litres/min.

The electromagnetic emission stressor is suitably applied by irradiating the aliquot under treatment from a source of an electromagnetic emission while the aliquot is maintained at the aforementioned temperature and while the oxygen/ozone gaseous mixture is being bubbled through the aliquot. Preferred electromagnetic emissions are selected from photonic radiation, more preferably UV, visible and infrared light, and even more preferably UV light. The most preferred UV sources are UV lamps emitting primarily UV-C band wavelengths, i.e. at wavelengths shorter than about 280 nm. Such lamps may also emit amounts of visible and infrared light. Ultraviolet light corresponding to standard UV-A (wavelengths from about 315 to about 400 nm) and UV-B (wavelengths from about 280 to about 315) sources can also be used. For example, an appropriate dosage of such UV light, applied simultaneously with the aforementioned temperature and oxidative environment stressors, can be obtained from up to eight lamps arranged to surround the sample container holding the aliquot, operated at an intensity to deliver a total UV light energy at the surface of the blood of from about 0.025 to about 10 joules/cm², preferably from about 0.1 to about 3.0 joules/cm², may advantageously be used. Preferably, four such lamps are used.

The time for which the aliquot is subjected to the stressors is normally within the time range of up to about 60 minutes. The time depends to some extent upon the chosen intensity of the electromagnetic emission, the temperature, the concentration of the oxidizing agent and the rate at which it is supplied to the aliquot. Some experimentation to establish optimum times may be necessary on the part of the operator, once the other stressor levels have been set. Under most stressor conditions, preferred times will be in the approximate range of from about 2 to about 5 minutes, more preferably about 3 minutes. The starting blood temperature, and the rate at which it can be warmed or cooled to a predetermined temperature, tends to vary from subject to subject. Such a treatment provides a modified blood aliquot which is ready for injection into the subject.

In the practice of the preferred process of the present invention, the blood aliquot may be treated with the stressors using an apparatus of the type described in U.S. Patent No. 4,968,483 to Mueller. The aliquot is placed in a suitable, sterile, UV light-transmissive container, which is fitted into the machine. The UV lamps are switched on for a fixed period before the gas flow is applied to the aliquot providing the oxidative stress, to allow the output of the UV lamps to stabilize. The UV lamps are typically on while the temperature of the aliquot is adjusted to the predetermined value, e.g. 42.5 ± 1 °C. Then the oxygen/ozone gas mixture, of known composition and controlled flow rate, is applied to the aliquot, for the predetermined duration of up to about 60 minutes, preferably 2 to 5 minutes and most preferably about 3 minutes as discussed above, so that the aliquot experiences all three stressors simultaneously. In this way, blood is appropriately modified according to the present invention to achieve the desired effects.

A subject may receive the blood aliquots with a course of daily administration for a number of consecutive days, or may comprise a first course of daily aliquots for a designated period of time, followed by an interval and then one or more additional courses of daily administration. In one preferred embodiment, each course comprises the administration of 4 to 6 daily aliquots. In another preferred embodiment, the preferred dosage regimen comprises the administration of from 2 to 4 aliquots of treated blood, with the administration of any pair of consecutive aliquots being either on consecutive days, or being separated by a rest period of from 1 to 21 days on which no aliquots are administered to the patient, the rest period separating one selected pair of consecutive aliquots being from about 3 to 15 days. A more specific, preferred dosage regimen would be a total of three aliquots, with the first and second aliquots being administered on consecutive days and a rest period of 11 days being provided, between the administration of the second and third aliquots. Preferably, successive courses of treatment are separated by a period of at least about three weeks during which no treated blood is administered to the subject. The spacing between successive courses of treatment should be such that the positive effects of the medical use of the invention are maintained. Similarly, the number of courses of administration should be such that the effects are maintained, and may be determined on the basis of the observed response of individual subjects.

The invention is further illustrated and described with reference to the following specific examples.

### EXAMPLE 1 - Animal Studies

### Model:

The purpose of the experiment is to determine the effects of treatment made possible with the present invention on the development of atherosclerosis in the LDL receptor (LDL-R) deficient mouse model, a widely used transgenic atherosclerosis model created by targeted disruption of the LDL receptor. This animal model is analogous to familial hypercholesterolemia, an inherited condition in which a mutation results in complete lack of functional LDL-R. In the human disease, homozygous individuals demonstrate a marked increase in serum cholesterol and develop severe premature atherosclerosis, often succumbing to this disease at an early age. In patients with this disease, currently used lipid lowering agents do not have a significant effect in terms of lowering cholesterol levels.

The LDL-R deficient mouse model shows intolerance to cholesterol feeding and develops widespread atherosclerotic changes which progress to mature fibrous lesions morphologically indistinguishable from established human atherosclerosis. Apart from the defined genetic abnormality causing predisposition to atherosclerosis, this model has the advantage of rapid development of widespread atherosclerosis within 6 to 8 weeks following institution of cholesterol feeding.

### Protocol:

LDL-R deficient mice were purchased from Jackson Laboratories. A total of 20 mice were entered into the study at 22 weeks of age, and 15 mice completed the study. The length of the study was 8 weeks. The mice were maintained on a 12 hour dark/12 hour light cycle with free access to food and water, and were fed a specified diet as follows. A control group comprised of 5 animals, all of which completed the study, received a normal diet. The high cholesterol group comprising 15 animals, of which 10 completed the study, were fed a diet containing 1.25% cholesterol. 7.5% cocoa butter, 7.5% casein, and 0.5% sodium cholate. To ensure proper food intake, food consumption and animal weight were monitored on a weekly basis. In previous experiments, it was demonstrated that 8 weeks of feeding with the high cholesterol diet results in substantial atherosclerosis development, particularly in the aortic arch and the descending thoracic aorta.

### Treatment:

Ten of the animals fed the high cholesterol diet were selected at random to undergo a course of treatment by the preferred embodiment of the invention. Six of the treated animals completed the study. It is to be noted that the four deaths in this group were not in any way related to the treatment, but occurred early in the study as a result of fighting among animals which were housed together during the study. The other five animals on the high cholesterol diet underwent a course of sham treatments. and four survived the protocol.

The treatments began four weeks after initiation of the study, with each of the animals on the high cholesterol diet receiving a total of 10 treatments (2 courses of treatment of 1 injection per day for 5 days, the 2 courses of treatment separated by two days, i.e. 10 injections over a period of 12 days). Each individual treatment administered to the animals consisted of the collection of 10 ml of blood from genetically compatible donor animals fed on a normal diet, the blood being collected into sodium citrate anticoagulant. In order to collect each 10 ml aliquot of blood, about 1 ml of blood was extracted from each of 10 animals. The blood was extracted by cardiac puncture, with the animals being under full xylazine/ketamine anesthesia during the blood extraction procedure, and being given T-61 immediately following extraction. The blood aliquot was transferred to a sterile, disposable, low-density polyethylene vessel for ex vivo treatment, and was then treated simultaneously with a gaseous oxygen/ozone mixture and ultraviolet light at elevated temperature using an apparatus as generally described in aforementioned U.S. Patent No. 4,968,483 to Mueller et al.

The constitution of the gas mixture was 14.5 ± 1.0 µg ozone/ml, with the remainder of the mixture comprising medical grade oxygen. The gas mixture was bubbled through the aliquot at a rate of 240 ± 24 ml/min for a period of 3 minutes. The temperature of the aliquot was held steady at 42.5 ± 1.0°C. The UV light was within the UV-C band, and included a wavelength of 253.7 nm.

After treatment by the preferred embodiment of the present invention, 30 µl of the treated blood was re-injected intramuscularly into each animal.

In the sham treatments, 30 µl of untreated blood was injected intramuscularly into each of the remaining five animals on the high cholesterol diet.

### Assessment of Atherosclerosis:

At sacrifice, the animals were anesthetized with zylaxine/ketamine and the heart was exposed. After nicking the vena cava to obtain blood samples, the animals were perfused via ventricular puncture, first with PBS to flush out the blood and then with 10% neutral buffered formalin for 3 minutes to fix the aorta. The thoracic aorta was dissected away from the thorax en bloc and stored in 10% formalin at 4°C. Pressure-fixed (10% formalin) aortae were removed en bloc and opened to allow a longitudinal full length inversion. The aortae were then mounted internally exposed on glass slides and stained with oil red O. The bright red staining (indicating lipid deposition) was then quantified using a computer assisted morphometric system, and expressed as the atherosclerotic index (ratio of atherosclerotic to normal, aortic surface area).

### Plasma Lipid and Lipoprotein Analysis:

Lipoprotein profiles were obtained by means of fast-phase liquid chromatography with a Superose 6B column. 200 µl aliquots of platelet-poor plasma from each animal were loaded onto the column and eluted with TSE buffer at a constant flow rate of 0.35 ml/min. An aliquot of 80 µl from each fraction was used for the measurement of total cholesterol minus esterified cholesterol. Total cholesterol and triglycerides in plasma samples and column fractions for 11 representative animals were measured by an enzymatic method established in the lipid research group at St. Michael's Hospital, Toronto.

### Statistical Analysis:

Continuous variables are reported as mean ± SD. Differences in cholesterol levels and triglyceride levels among groups were tested by Student's t-test. Differences in atherosclerotic lesion area among groups were tested using the one-way ANOVA test in conjunction with the Bonferroni correction.

### Results:

Figure 1 illustrates two full length aortae stained with oil red O to detect lipid deposition and plaque formation inside the arteries. The animals which received the high cholesterol diet and the sham treatments exhibited substantial aortic lipid deposition (aorta 1 in Figure I ), with a ratio of atherosclerotic area (AA) to total area (TA) being 0.16 ± 0.1. In comparison, those animals which were treated by the preferred method of the present invention showed a profoundly reduced level of aortic lipid deposition (aorta 2 in Figure 1), with AA/TA being 0.04 ± 0.02. These ratios are significantly different, with p < 0.05. In the animals which received the normal diet, no significant atherosclerotic changes were observed.

In addition, the animals which were treated according to the preferred method of the present invention were observed to have better general appearance, reduced skin xanthomatosis (eyelids, nose and paws), reduced limb swelling, and better appetite than the untreated animals which received the high cholesterol diet.

To further illustrate the effects of the medical use of the present invention. sections of aortae obtained from animals in this example were immunostained for MOMA-2, a marker of inflammatory monocyte/macrophages. These results are illustrated in Figures 2 (normal diet), 3 (HC diet - no treatment) and 4 (HC diet - treated). No staining is seen in Figure 2. Staining is visible in both Figures 3 and 4, indicating that the majority of cells in the neointima of the animals which received the high cholesterol diet were monocyte/macrophages. However, as seen by comparing Figures 3 and 4, animals that had received the blood aliquots according to the claimed medical use showed a marked reduction in the amount of plaque deposition, and therefore also show a decrease in the overall number of monocyte/macrophages. This indicates a reduction in inflammatory processes in the plaque of treated compared to control animals. Furthermore, it is believed that inflammatory cells present in the plaque are largely responsible for plaque instability, which is believed to be the primary cause of thrombus formation. It is expected that the increased plaque stability produced by the method of the invention will bring about a corresponding decrease in cardiovascular event rates.

The measurement of the cholesterol levels among the different groups of animals showed that the total serum cholesterol level did not change significantly in the animals which received the normal diet, but was markedly increased in the animals receiving the high cholesterol diet. This marked increase occurred both in the sham treated group and to a lesser extent in the group which received the treatments according to the preferred method of the present invention.

The measured triglyceride levels were also higher in the animals which received the high cholesterol diet, as compared to the animals which received the normal diet. However, among the animals which received the high cholesterol diet, the increase in triglyceride levels was much greater in the sham treated group than in the group which was treated according to the preferred method of the present invention.

The measured cholesterol (total and HDL) and triglyceride levels, and the average cholesterol and triglyceride levels for each group, are shown below in Table 1.

**Table 1**

| **GROUP** | **ANIMAL** | **Total Cholesterol** **(mM)** | **HDL Cholesterol** **(mM)** | **Triglycerides** **(mM)** |
|---|---|---|---|---|
| Treated | 1 | 13.0 | 0.84 | 0.30 |
| Treated | 2 | 13.2 | 0.81 | 0.34 |
| Treated | 3 | 16.2 | 0.89 | 0.35 |
| Treated | 4 | 14.9 | 1.10 | 0.40 |
| | **Average (1 - 4)** | **14.3 ± 1.5** | **0.91 ± 0.13** | **0.35 ± 0.04** |
| Control | 6 | 4.4 | 1.02 | 0.34 |
| Control | 7 | 5.2 | 1.09 | 0.40 |
| Control | 8 | 5.7 | 1.44 | 0.44 |
| | **Average (6-8)** | **5.1 ± 0.7** | **1.18 ± 0.23** | **0.39 ± 0.05** |
| HC Diet | 11 | 27.5 | 0.87 | 0.70 |
| HC Diet | 12 | 26.6 | 0.90 | 0.72 |
| HC Diet | 13 | 23.4 | 0.78 | 0.60 |
| HC Diet | 14 | 24.6 | 1.23 | 0.64 |
| | **Average (11 - 14)** | **25.5 ± 1.9** | **0.95 ± 0.20** | **0.66 ± 0.05** |

In Table 1, the "Treated" group of animals received the high cholesterol diet and were treated according to the preferred method of the present invention, the "Control" animals received the normal diet and no treatment, and the "HC Diet" animals received the high cholesterol diet and the sham treatment.

### EXAMPLE 2 - Human Studies

The reduction in triglyceride levels observed in the animal studies is consistent with results obtained in two human studies, the results of which are presented below.

### Human Study A

A total of 94 healthy human volunteers, of which 57 were males and 37 were females, were given a course of six aliquots according to the preferred embodiment of the present invention over a two week period. Serum triglyceride levels were measured before and after the course of treatments, and the average measurements are shown below in Table 2.

**Table 2**

| Pre-treatment triglyceride level (mg/dl) | Post-treatment triglyceride level (mg/dl) | Significance (p) |
|---|---|---|
| 146 | 134 | 0.04 |

### Human Study B

A total of 49 patients from family practice were given various schedules of administration according to the preferred embodiment of the present invention. The schedules comprised from 5 to 40 injections per patient. Serum triglyceride levels of each patient were measured before and after the course of therapy and the average measurements are presented below in Table 3.

**Table 3**

| Pre-treatment triglyceride level (mg/dl) | Post-treatment triglyceride level (mg/dl) | Significance (p) |
|---|---|---|
| 135.4 | 121.9 | < 0.05 |

From the above human studies, it can be concluded that the medical use according to the preferred embodiment of the present invention achieves a significant reduction in total serum triglyceride levels in human subjects.

### EXAMPLE 3 - Animal Studies

In this study, LDL-R deficient mice were divided into groups and studied using the following protocol:
Group A (control) - fed a normal diet as in Example 1;
Group B1 - fed a high cholesterol diet as described in Example 1 for 8 weeks;
Group B2 - fed a high cholesterol diet as described in Example 1 for 12 weeks;
Group C1 - fed a high cholesterol diet as described in Example 1 for 8 weeks, and treated by the preferred method of the present invention as described in Example 1 at 4 weeks of dietary intervention; and
Group C2 - fed a high cholesterol diet as described in Example 1 for 12 weeks, and treated by the preferred method of the present invention as described in Example I at 8 weeks of dietary intervention.

For each group of animals, the atherosclerotic index was assessed at either 8 or 12 weeks according to the method described in Example 1 under the heading "Assessment of Atherosclerosis". As demonstrated by measurement of atherosclerotic area, the animals of group B (high cholesterol diet alone) exhibited substantial aortic lipid deposition, with group B1 animals having an atherosclerotic index of 0.16 ± 0.1 at eight weeks and group B2 animals having an index of 0.17 ± 0.1 at 12 weeks of dietary intervention. In contrast, the animals of group C (high cholesterol diet with treatment according to the invention) exhibited profoundly reduced lipid deposition, with group C1 animals having an index of 0.04 ± 0.02 (p < 0.05) at eight weeks of dietary intervention, and group C2 animals having levels of 0.04 ± 0.02 (p < 0.01) at twelve weeks of dietary intervention.

The animals of group C also exhibited a marked reduction in xanthelasma and limb swelling as compared to animals of group B.

Total lipoprotein profiles were measured as in Example 1 by fast-phase liquid chromatography and an enzyme-linked assay. The results of this analysis showed that the animals of group B (high cholesterol diet alone) had markedly increased levels of total serum cholesterol (CHO 46.71 ± 3.61 mM) as compared to control group A (CHO 5.1 ± 0.7 mM). The animals of group C did not show a significant reduction in cholesterol (CHO 44.7 ± 2.8 mM; p = 0.27 for B vs. C) as compared to the animals of group B.

As shown in the above examples, the claimed medical use substantially inhibited the development of atherosclerosis in a mouse model of human familial hypercholesterolemia. In addition to substantially reducing the development of atherosclerosis at an early stage and inhibiting the progression of established atherosclerotic lesions, said use was shown to cause regression of existing atherosclerotic lesions. This can be seen for example by comparing the results for the animals of sub-groups B1 and C2 in Example 3, which show that existing plaque deposits at week eight of a high cholesterol diet are reduced by about 75 % when the animals are treated at week eight according to the present invention. These improvements in cardiovascular health were accompanied by improvements in the animals' general overall appearance and appetite.

As discussed above, atherosclerosis has a significant immune-modulated inflammatory component. It is therefore believed that the ability of the method of the invention to prevent and treat atherosclerosis is at least partially due to its anti-inflammatory action, and in particular its ability to produce an increase in the TH2 cells and/or a decrease in TH1 cells in the blood of treated subjects.

## Claims

1. Use in preparation of a medicament for preventing progression of atherosclerosis in a mammal, of an aliquot of the mammal's blood which has been subjected extracorporeally to an electromagnetic emission and an oxidative environment.

2. Use according to claim 1, wherein the oxidative environment comprises applying an oxidizing agent to the aliquot.

3. Use according to claim 2, wherein the oxidizing agent contains ozone gas, and the ozone gas is introduced into the blood aliquot in an amount which does not give rise to excessive levels of mutagenicity.

4. Use according to claim 2, wherein the oxidizing agent comprises a mixture of ozone gas and medical grade oxygen, the ozone gas being contained in the mixture in a concentration of up to 300 µg/ml.

5. Use according to claim 4, wherein the ozone gas is contained in the mixture in a concentration of up to 30 µg/ml.

6. Use according to claim 4, wherein the mixture is applied to the aliquot at a flow rate of up to 0.33 litres/min.

7. Use according to claim 1, wherein the electromagnetic emission comprises ultraviolet light having one or more UV-C band wavelengths.

8. Use according to claim 1, wherein the aliquot is further subjected to a temperature stressor, said temperature stressor comprising a temperature above or below body temperature which does not result in substantial hemolysis of the blood in the aliquot.

9. Use according to claim 8, wherein the temperature stressor is applied so that the temperature of at least part of the aliquot is in the range of from -5°C to 55°C.

10. Use according to claim 9, wherein the mean temperature of the blood in the aliquot is in the range of from 37°C to 44°C.

11. Use according to claim 9, wherein the mean temperature of the blood in the aliquot is in the range of from 0°C to 36.5°C.

12. Use according to claim 9, wherein the mean temperature of the blood in the aliquot is in the range of from 10°C to 30°C.

13. Use according to claim 9, wherein the mean temperature of the blood in the aliquot is in the range of from 37°C to 55°C.

14. Use according to claim 13, wherein the temperature is 42.5 ± 1°C.

15. Use according to claim 1, wherein the aliquot has a volume of up to 400 ml.

16. Use according to claim 15, wherein the volume of the aliquot is 10 ml.

17. Use according to claim 1, wherein the aliquot is subjected to the electromagnetic emission and the oxidative environment for a period of up to 60 minutes.

18. Use according to claim 17, wherein the aliquot is subjected to the electromagnetic emission and the oxidative environment for a period of 3 minutes.

19. Use according to claim 8, wherein the aliquot is simultaneously subjected to the electromagnetic emission, the oxidative environment and the temperature stressor.

20. Use according to claim 1, wherein the aliquot is simultaneously subjected to the electromagnetic emission and the oxidative environment.

## Patentansprüche

1. Verwendung bei der Herstellung eines Arzneimittels zur Verhinderung des Fortschreitens von Atherosklerose bei einem Säuger eines Aliquots des Säugerblutes, das extrakorporal einer elektromagnetischen Emission und einer oxidativen Umgebung unterworfen worden ist.

2. Verwendung nach Anspruch 1, **dadurch gekennzeichnet, daß** die oxidative Umgebung das Beaufschlagen des Aliquots mit einem Oxidationsmittel umfaßt.

3. Verwendung nach Anspruch 2, **dadurch gekennzeichnet, daß** das Oxidationsmittel Ozongas enthält und das Ozongas in das Blutaliquot in einer Menge eingebracht wird, die nicht zu unangemessen hohen Mutagenitätsniveaus führt.

4. Verwendung nach Anspruch 2, **dadurch gekennzeichnet, daß** das Oxidationsmittel eine Mischung aus Ozongas und Sauerstoff medizinischer Qualität umfaßt, wobei das Ozongas in der Mischung in einer Konzentration von bis zu 300 µg/ml enthalten ist.

5. Verwendung nach Anspruch 4, **dadurch gekennzeichnet, daß** das Ozongas in der Mischung in einer Konzentration von bis zu 30 µg/ml enthalten ist.

6. Verwendung nach Anspruch 4, **dadurch gekennzeichnet, daß** das Aliquot mit der Mischung mit einer Durchflußrate von bis zu 0,33 Litern/min beaufschlagt wird.

7. Verwendung nach Anspruch 1, **dadurch gekennzeichnet, daß** die elektromagnetische Emission ultraviolettes Licht mit einer oder mehreren Wellenlängen im UV-C-Band umfaßt.

8. Verwendung nach Anspruch 1, **dadurch gekennzeichnet, daß** das Aliquot weiter einem Temperaturstressor unterworfen wird, wobei besagter Temperaturstressor eine Temperatur oberhalb oder unterhalb Körpertemperatur umfaßt, die nicht zu wesentlicher Hämolyse des Blutes im Aliquot führt.

9. Verwendung nach Anspruch 8, **dadurch gekennzeichnet, daß** der Temperaturstressor so angewendet wird, daß die Temperatur wenigstens eines Teils des Aliquots im Bereich von -5°C bis 55°C liegt.

10. Verwendung nach Anspruch 9, **dadurch gekennzeichnet, daß** die mittlere Temperatur des Blutes im Aliquot im Bereich von 37°C bis 44°C liegt.

11. Verwendung nach Anspruch 9, **dadurch gekennzeichnet, daß** die mittlere Temperatur des Blutes im Aliquot im Bereich von 0°C bis 36,5°C liegt.

12. Verwendung nach Anspruch 9, **dadurch gekennzeichnet, daß** die mittlere Temperatur des Blutes im Aliquot im Bereich von 10°C bis 30°C liegt.

13. Verwendung nach Anspruch 9, **dadurch gekennzeichnet, daß** die mittlere Temperatur des Blutes im Aliquot im Bereich von 37°C bis 55°C liegt.

14. Verwendung nach Anspruch 13, **dadurch gekennzeichnet, daß** die Temperatur 42,5 ± 1°C beträgt.

15. Verwendung nach Anspruch 1, **dadurch gekennzeichnet, daß** das Aliquot ein Volumen von bis zu 400 ml hat.

16. Verwendung von Anspruch 15, **dadurch gekennzeichnet, daß** das Volumen des Aliquots 10 ml beträgt.

17. Verwendung nach Anspruch 1, **dadurch gekennzeichnet, daß** das Aliquot der elektromagnetischen Emission und der oxidativen Umgebung für einen Zeitraum von bis zu 60 Minuten unterworfen wird.

18. Verwendung nach Anspruch 17, **dadurch gekennzeichnet, daß** das Aliquot der elektromagnetischen Emission und der oxidativen Umgebung für einen Zeitraum von 3 Minuten unterworfen wird.

19. Verwendung nach Anspruch 8, **dadurch gekennzeichnet, daß** das Aliquot gleichzeitig der elektromagnetischen Emission, der oxidativen Umgebung und dem Temperaturstressor unterworfen wird.

20. Verwendung nach Anspruch 1, **dadurch gekennzeichnet, daß** das Aliquot gleichzeitig der elektromagnetischen Emission und der oxidativen Umgebung unterworfen wird.

## Revendications

1. Utilisation dans une préparation d'un médicament pour prévenir la progression de l'athérosclérose chez un mammifère, d'une aliquote du sang d'un mammifère, que l'on a soumis de façon extra-corporelle à une émission électromagnétique et à un environnement oxidatif.

2. Utilisation selon la revendication 1, dans lequel l'environnement oxidatif comprend l'application d'un agent oxydant à l'aliquote.

3. Utilisation selon la revendication 2, dans laquelle l'agent oxydant contient du gaz ozone, et dans laquelle on introduit le gaz ozone dans l'aliquote de sang en une quantité n'induisant pas des niveaux excessifs de mutagénicité.

4. Utilisation selon la revendication 2, dans laquelle l'agent oxydant comprend un mélange de gaz ozone et d'oxygène de qualité médicale, le gaz ozone étant contenu dans le mélange à une concentration allant jusqu'à 300 µg /ml.

5. Utilisation selon la revendication 4, dans laquelle le gaz ozone est contenu dans le mélange à une concentration allant jusqu'à 30 µg/ml.

6. Utilisation selon la revendication 4, dans laquelle on applique le mélange à l'aliquote à un débit allant jusqu'à 0,33 litres/min.

7. Utilisation selon la revendication 1, dans laquelle l'émission électromagnétique comprend une lumière ultraviolette comportant une ou plusieurs longueurs d'ondes dans la bande UV-C.

8. Utilisation selon la revendication 1, dans laquelle on soumet ensuite l'aliquote à un inducteur de température, ledit inducteur de température comprenant une température au-dessus ou en dessous de la température corporelle, ne résultant pas en une hémolyse substantielle du sang de l'aliquote.

9. Utilisation selon la revendication 8, dans laquelle on applique l'inducteur de température de sorte que la température d'au moins une partie de l'aliquote soit dans une fourchette allant de -5°C à 55°C.

10. Utilisation selon la revendication 9, dans laquelle la température moyenne du sang dans l'aliquote est dans la fourchette allant de 37°C à 44°C.

11. Utilisation selon la revendication 9, dans laquelle la température moyenne du sang dans l'aliquote est dans la fourchette allant de 0°C à 36,5°C.

12. Utilisation selon la revendication 9, dans laquelle la température moyenne du sang dans l'aliquote est dans la fourchette allant de 10°C à 30°C.

13. Utilisation selon la revendication 9, dans laquelle la température moyenne du sang dans l'aliquote est dans la fourchette allant de 37°C à 55°C.

14. Utilisation selon la revendication 13, dans laquelle la température est de 42,5 ± 1°C.

15. Utilisation selon la revendication 1, dans laquelle l'aliquote présente un volume allant jusqu'à 400 ml.

16. Utilisation selon la revendication 15, dans laquelle le volume de l'aliquote est de 10 ml.

17. Utilisation selon la revendication 1, dans laquelle on soumet l'aliquote à une émission électromagnétique et à un environnement oxidatif pour une période allant jusqu'à 60 minutes.

18. Utilisation selon la revendication 17, dans laquelle on soumet l'aliquote à une émission électromagnétique et à un environnement oxidatif pour une période de 3 minutes.

19. Utilisation selon la revendication 8, dans laquelle on soumet simultanément l'aliquote à une émission électromagnétique, un environnement oxidatif et un inducteur de température.

20. Utilisation selon la revendication 1, dans laquelle on soumet simultanément l'aliquote à une émission électromagnétique et à un environnement oxidatif.
